# EUROPÄISCHE PATENTSCHRIFT

(11) **EP 3 886 770 B1**
(45) Veröffentlichungstag und Bekanntmachung des Hinweises auf die Patenterteilung: **19.07.2023**
(21) Anmeldenummer: 19809427.8
(22) Anmeldetag: 22.11.2019
(51) Int. Cl.: A61F 2/78

(54) **PROTHESENLINER**
PROSTHETIC LINER
MANCHON PROTHÉTIQUE

(30) Priorität: 26.11.2018 DE 102018129737
(43) Veröffentlichungstag der Anmeldung: 06.10.2021
(73) Patentinhaber: Ottobock SE & Co. KGaA, 37115 Duderstadt (DE)
(72) Erfinder: HILLMANN, Martin, 37115 Duderstadt (DE); GARUS, Bernard, 37574 Einbeck (DE)
(74) Vertreter: Gramm, Lins & Partner Patent- und Rechtsanwälte PartGmbB
(86) Internationale Anmeldenummer: PCT/EP2019/082241
(87) Internationale Veröffentlichungsnummer: WO 2020/109166

(56) Entgegenhaltungen:
- EP-B1- 2 538 892
- WO-A1-2014/032815
- US-A1- 2005 149 202
- US-A1- 2005 240 283
- US-A1- 2007 123 998
- US-A1- 2015 057 763

## Beschreibung

Die Erfindung betrifft einen Prothesenliner mit einem Grundkörper aus einem elastischen Linermaterial, der ein offenes proximales Ende, ein dem proximalen Ende entgegen gesetztes geschlossenes distales Ende und eine Längserstreckung aufweist, die sich von dem proximalen Ende zu dem distalen Ende erstreckt, einer bielastischen Textillage, die an oder in dem Linermaterial angeordnet ist, und einem Verbindungsbereich, der angeordnet und eingerichtet ist, mit einem Prothesenschaft verbunden zu werden.

Derartige Prothesenliner sind aus dem Stand der Technik seit langem bekannt. Prothesenliner werden als Zwischenlage zwischen einem Restglied des Trägers der Prothese, das auch als Amputationsstumpf bezeichnet wird, und einem Prothesenschaft verwendet, an dem die eigentliche künstliche Gliedmaße angeordnet ist. Insbesondere bei Beinprothesen aber auch bei Armprothesen ist es sowohl für den Tragekomfort als auch für die gefühlte Sicherheit des Trägers von größter Bedeutung, eine feste Verbindung zwischen den Amputationsstumpf und dem sich darüber befindenden Prothesenliner und der weiteren Prothese, insbesondere also dem Prothesenschaft, herbeizuführen, ohne dass es dabei zu Druckstellen, schmerzhaften Wunden oder einem Scheuern eines der Bauteile an der Haut des Trägers kommt.

Prothesenliner werden im Wesentlichen in zwei unterschiedlichen Ausführungsformen verwendet.

Es gibt Prothesenliner, die über den Amputationsstumpf gezogen werden, so dass ein Volumen zwischen dem Prothesenschaftes und dem Prothesenliner, der über den Amputationsstumpf gezogen wurde, luftdicht abgeschlossen wird, indem der Prothesenliner mit dem Amputationsstumpf in den Prothesenschaft eingeführt wird. Über eine Pumpe oder durch ein Ausstoßventil kann in diesem vorzugsweise hermetisch abgeschlossenen Volumen ein Unterdruck erzeugt werden, durch den der Prothesenschaft am Amputationsstumpf, der sich in dem Liner befindet, gehalten wird. Ein passives Ausstoßventil ist dabei bevorzugt derart angeordnet und ausgebildet, dass es beim Gehen mit einer Prothese, die an dem Prothesenschaft angeordnet ist, betätigt wird. Insbesondere in der Schwungphase eines Gangzyklus entstehen große Kräfte, die auf den Prothesenschaft in distale Richtung wirken. Diese werden durch den Unterdruck relativ gleichmäßig auf den Prothesenliner und damit auf den sich darin befindenden Amputationsstumpf übertragen. In diesem Fall ist der Verbindungsbereich des Prothesenliners der Bereich, der das Volumen, in dem der Unterdruck hergestellt wird, begrenzt. Der Verbindungsbereich erstreckt sich in der Regel von dem distalen Ende des Prothesenliners nach proximal. In einigen Ausführungsformen des Prothesenliners ist an einer Außenseite des Grundkörpers wenigstens eine, gegebenenfalls auch mehrere, Dichtlippen angeordnet. Durch diese wird das Volumen, in dem der Unterdruck hergestellt wird und damit auch der Verbindungsbereich nach proximal begrenzt.

Eine alternative Ausführungsform des Liners verfügt insbesondere an seinem distalen Ende über eine Kappe oder Tasse, an der ein Befestigungselement angeordnet ist, das beispielsweise in Form eines distal hervorstehenden Dorns ausgebildet sein kann. Dieses Befestigungselement wird verwendet, um mit einem korrespondierend ausgebildeten Befestigungselement im Innern des Prothesenschaftes zusammenzuwirken und auf diese Weise eine möglichst sichere Verbindung zwischen dem Prothesenliner und dem Prothesenschaft herzustellen. Bei dieser Art der Verbindung zwischen Prothesenschaft und Prothesenliner werden auftretende Zugkräfte, die vom Prothesenschaft auf den Prothesenliner übertragen werden müssen, nahezu vollständig durch die Befestigungselemente übertragen. Die auftretenden Zugkräfte wirken folglich nahezu ausschließlich auf den distalen Bereich des Liners, wodurch einerseits dieser Bereich auch mechanisch stark belastet wird und andererseits es insbesondere in diesem Bereich zu dem für den Träger unbequemen Melkeffekt am Amputationsstumpf aufgrund der stark wechselnden Belastung kommt. In diesem Fall ist der Verbindungsbereich auf die distale Tasse oder Kappe begrenzt.

Dieser Effekt wird verstärkt, da das Linermaterial, aus dem der Grundkörper des Prothesenliners hergestellt ist, elastisch ist. Es handelt sich beispielsweise um ein Silikon, ein thermoplastisches Elastomer (TPE) oder ein Polyurethan, das sowohl in Längserstreckung des Grundkörpers als auch in Umfangsrichtung des Grundkörpers elastisch ausgebildet ist. Diese Elastizität ist notwendig, um für unterschiedliche Größen und Formen von Amputationsstümpfen passende Standardliner herstellen zu können. Zudem ist es notwendig, den Liner beim Anziehen oder Anlegen an den Amputationsstumpf aufzuweiten, um die benötigte Haltekraft gewährleisten zu können.

Aus der EP 2 538 892 B1, die den Oberbegriff des Anspruchs 1 offenbart, ist ein Prothesenliner bekannt, dessen Textillage aus zwei unterschiedlichen Textilen zusammengesetzt ist. Im Bereich eines Gelenkes, beispielsweise eines Knies, das im angelegten Zustand von dem Prothesenliner überdeckt und umschlossen wird, ist ein erstes Textil angeordnet, das aus einem bielastischen Material besteht. Bielastisch bedeutet dabei, dass sowohl in Längserstreckung als auch in Umfangsrichtung des Grundkörpers das Material elastisch ausgebildet ist. In allen anderen Bereichen ist die Textillage aus einem anderen Textil hergestellt, das monoelastisch ausgebildet ist. Eine Elastizität in Längserstreckung des Grundkörpers ist stark eingeschränkt, in Umfangsrichtung ist die Elastizität jedoch vorhanden.

Nachteilig ist, dass die Herstellung eines derartigen Prothesenliners aufgrund der Mehrzahl zu verwenden Textilelemente, die miteinander vernäht werden müssen, bevor sie mit dem Grundkörper verbunden werden, aufwendig und daher kostenintensiv ist. Zudem kann es durch die notwendigerweise vorhandenen Nähte, die die einzelnen Textilelemente miteinander verbinden, zu Druckstellen oder reduziertem Tragekomfort kommen.

Aus der US 2005/0149202 A1 ist ein Liner bekannt, der einen strumpfförmigen Grundkörper aus einem Gewebe aufweist. Im Bereich der distalen Kappe ist eine Beschichtung auf die Außenseite dieses Grundkörpers aufgetragen, die eine geringere Elastizität als das Gewebe aufweist. In einer anderen gezeigten Ausgestaltung werden statt der Beschichtung mehrere Streifen aus einem Gewebe oder Textil aufgebracht, das gegenüber dem Gewebe des Grundkörpers eine reduzierte Elastizität aufweist. Auch hier kommt es aufgrund der mehreren Textilelemente, die miteinander verbunden werden müssen, zu Nähten, die die bereits genannten Nachteile aufweisen.

Die US 6,231,617 B1 beschreibt einen Liner aus einem elastischen Kunststoff, beispielsweise einem Urethan oder aus Silikon. In diesem Material sind mehrere Arme aus einem in Längsrichtung inelastischen Material eingebettet, die beispielsweise aus einem Metall, einem Kunststoff, Baumwolle oder Kohlefasern hergestellt sein können. Ein ähnliches Prinzip ist der US 4,923,474 B1 zu entnehmen. Auch hier werden in einem Grundkörper aus einem elastischen Material, beispielsweis einem Gummi-Material, inelastische Elemente eingebettet, die die Elastizität des Liners in Längsrichtung verringern.

Aus der US 2005/149202 A1 und der US 2005/240283 A1 sind jeweils Liner bekannt, deren Gel- oder Silikonkörper mit einer vorzugsweise auf der Außenseite angeordneten Textilschicht versehen sind. In der US 2015/0057736 A1 hat diese Textilschicht in unterschiedlichen Bereichen unterschiedliche Elastizitäten. Die US 2007/0123998 A1 befasst sich mit Linern, an deren Außenseite unterschiedlich geformte Dichtlippen angeordnet sind, die distale Volumen zwischen dem Liner und dem Schaft abdichten sollen.

Die WO 2014/032815 A1 beschreibt einen Liner, der zwei sich kreuzende Lagen von Fasern oder ein entsprechendes Fasergeflecht ausweist. Dadurch wird die eine Änderung der Länge des Liners mit einer Änderung des Umfanges gekoppelt, so dass es nicht mehr möglich ist, den Liner beispielsweise durch eine Zugkraft zu verlängern, ohne ihn gleichzeitig in seinem Umfang zu verringern. Dies ist ansonsten bei elastischen Materialien, die als Linermaterial verwendet werden, möglich.

Der Erfindung liegt daher die Aufgabe zugrunde, einen Prothesenliner gemäß dem Oberbegriff des Anspruchs 1 so zu verbessern, dass die genannten Nachteile vermieden oder zumindest vermindert werden und der Liner einfach und kostengünstig herstellbar ist.

Die Erfindung löst die gestellte Aufgabe durch einen Prothesenliner gemäß dem Oberbegriff des Anspruchs 1, der sich dadurch auszeichnet, dass die Textillage in einem Abschnitt, der sich von dem Verbindungsbereich nach proximal erstreckt, wenigstens ein Zugelement aufweist, durch das die Elastizität der Textillage in Textilrichtung des Grundkörpers reduziert ist und das Fasern und/oder Fäden aus einem inelastischen Material aufweist.

Bevorzugt erstreckt sich der Abschnitt, in dem die Textillage über das wenigstens eine Zugelement verfügt, vorzugsweise von dem distalen Ende ausgehend. Dabei wird der Verbindungsbereich vorzugsweise durch eine distale Kappe mit wenigstens einem Befestigungselement zum Befestigen des Prothesenliners an einem Prothesenschaft gebildet.

Alternativ oder zusätzlich dazu verfügt der Prothesenliner über wenigstens eine Dichtlippe, durch die der Verbindungsbereich nach proximal begrenzt wird. Die Dichtlippe ist vorzugsweise derart angeordnet und ausgebildet, dass sie im angelegten Zustand des Liners mit einer Innenwand eines Prothesenschaftes in Kontakt kommt, wenn der Amputationsstumpf, über den der Prothesenliner gezogen wurde, in den Prothesenschaft eingeführt wird. Der Kontakt zwischen der Innenwand des Prothesenschaftes und der Dichtlippe sorgt dann bevorzugt dafür, dass ein sich distal der Dichtlippe erstreckendes Volumen luftdicht abgeschlossen wird, so dass darin der benötigte Unterdruck hergestellt werden kann.

In einer bevorzugten Ausgestaltung der Erfindung erstreckt sich das wenigstens eine Zugelement in den Verbindungsbereich hinein. Es handelt sich dann um das gleiche Zugelement, das sich in den Verbindungsbereich hinein und von dem Verbindungsbereich nach proximal erstreckt. Alternativ oder zusätzlich dazu weist die Textillage zusätzlich auch in dem Verbindungsbereich wenigstens ein Zugelement auf, durch das die Elastizität der Textillage in Längsrichtung des Grundkörpers reduziert wird

Vorzugsweise verfügt die Textillage über eine Mehrzahl von Zugelementen, die besonders bevorzugt äquidistant über einen Umfang der Textillage verteilt angeordnet sind. In einer alternativen Ausgestaltung sind die Zugelemente ausschließlich in der neutralen Faser des Prothesenliners angeordnet. Herkömmlicherweise werden Prothesenliner so ausgebildet, dass sie ein Gelenk des Trägers des Prothesenliners, beispielweise ein Kniegelenk umschließen. Wird das Gelenk gebeugt, wird ein Teil des umschließenden Prothesenliners gestreckt und ein anderer Teil gestaucht. Bei einem Kniegelenk wird bei Beugen des Gelenks (Flexion) der vordere Teil des Prothesenliners (ventral) gestreckt und der hintere Teil (dorsal) gestaucht. Es gibt auch Bereiche, die weder gestreckt noch gestaucht werden. Diese befinden sich in diesem Beispiel medial und lateral des Gelenkes und werden als "neutrale Faser" bezeichnet.

Diese Ausgestaltung ist insbesondere, jedoch nicht ausschließlich, für Prothesenliner von Vorteil, die in ihrem distalen Bereich Befestigungselemente oder sonstige Verbindungseinrichtungen aufweisen, um den Prothesenliner in diesem Bereich mit einem Prothesenschaft oder einem anderen Prothesenelement zu verbinden. Auftretende Zugkräfte, die beispielsweise in der Schwungphase eines Schrittzyklus auftreten können, werden durch die Verbindungselemente und Befestigungselemente in den distalen Bereich des Prothesenliners und der Textillage eingeleitet. Sie wirken in Längserstreckung des Prothesenliners. Die Elastizität des Linermaterials wird dabei in dem Abschnitt, der sich von distalen Ende der Textillage ausgehend erstreckt durch die Verwendung des wenigstens einen Zugelementes stark eingeschränkt oder vollständig aufgehoben. In dem Bereich, in dem beispielsweise in der Schwungphase eines Schrittes Zugkräfte in den Prothesenliner und damit in die Textillage eingeleitet werden, ist sie in dieser Richtung inelastisch, so dass die Zugkräfte über das wenigstens eine Zugelement weitergeleitet und in einem größeren Bereich, nämlich insbesondere einen Teil des Abschnittes, in dem sich das wenigstens eine Zugelement befindet, verteilt. Dadurch kommt es zu einer gleichmäßigeren Verteilung der auftretenden Kräfte und damit zu einer Reduzierung des auf den Amputationsstumpf wirkenden Druckes. Zudem wird auch dieser gleichmäßiger verteilt, wodurch der Tragekomfort erhöht wird.

Erfindungsgemäß verfügen die Zugelemente über Fasern und/oder Fäden aus einem inelastischen Material. So kann beispielsweise ein inelastisches Garn verwendet werden, das in das Textil eingelegt und bereits bei der Herstellung des Textils eingearbeitet wird. Die Fäden erstrecken sich vorteilhafterweise parallel zur Längserstreckung, beginnen also am distalen Ende der Textillage und erstrecken sich in Richtung auf das proximale Ende des Grundkörpers. Vorzugsweise ist die Textillage auf einer Außenseite des Grundkörpers angeordnet. Die Textillage deckt vorteilhafterweise die gesamte Außenseite des Grundkörpers ab, so dass auch das offene proximale Ende des Grundkörpers von einem ebenso offenen proximalen Ende der Textillage umgeben ist. In Richtung auf diese proximale Ende erstrecken sich vorteilhafterweise die Zugelemente, die am distalen Ende der Textillage beginnen. Durch die Verwendung eines inelastischen Materials als Zugelement, kann die Elastizität der Textillage in dieser Richtung nahezu vollständig oder sogar vollständig entfernt werden. Die Textillage ist dann in Richtung der Längserstreckung inelastisch. Da die Textillage möglichst vollflächig mit dem Grundkörper aus dem elastischen Linermaterial verbunden ist, kann auch der Grundkörper in diesem Bereich in Richtung seiner Längserstreckung nicht gedehnt werden, so dass die Elastizität des Grundkörpers entlang seiner Längserstreckung in diesem Abschnitt, der sich vom distalen Ende ausgehend erstreckt, stark reduziert oder sogar vollständig entfernt wurde.

In einer bevorzugten Ausgestaltung sind die Zugelemente an ihrem jeweiligen proximalen Ende an der Textillage befestigt. Auf diese Weise können auf die Zugelemente wirkende Zugkräfte auf die Textillage weitergegeben und übertragen werden. Das proximale Ende der Zugelemente ist bevorzugt vom proximalen Ende des Grundkörpers beabstandet. Dies bedeutet, dass die Zugelemente sich nicht über die vollständige Längserstreckung der Textillage und damit auch nicht über die vollständige Längserstreckung des Grundkörpers erstrecken. Der Prothesenliner verfügt folglich über den bereits genannten Abschnitt, der sich von seinem distalen Ende ausgehend erstreckt, in dem eine Elastizität des Prothesenliners entlang der Richtung der Längserstreckung stark reduziert oder sogar vollständig entfernt wurde und einem restlichen Anteil, in dem sich die Zugelemente nicht erstrecken, indem die ursprüngliche Elastizität der bielastischen Textillage und des elastischen Linermaterials weiterhin gegeben ist. Insbesondere ist in diesem restlichen Anteil des Prothesenliners auch eine Elastizität entlang der Längserstreckung vorhanden.

Vorzugsweise verfügen die Zugelemente an ihrem proximalen Ende über eine Schlaufe, die um wenigstens ein Element der Textillage herumgeführt ist. Handelt es sich bei dem Textil beispielsweise um ein Gewebe, wird die Schlaufe vorzugsweise um wenigstens einen Schussfaden und/oder wenigstens einen Kettfaden herumgeführt.

Bevorzugte Zugelement werden aus einem Faden oder Garn gebildet, das vorzugsweise inelastisch ist. Dieser Faden verfügt über zwei Enden und wird zur Bildung des Zugelementes vorteilhafterweise doppelt gelegt, so dass seine beiden Enden ein Ende des Zugelementes bilden und eine Schlaufe das entgegengesetzte Ende. Das durch die Schlaufe gebildete Ende bildet das proximale Ende des Zugelementes und wird vorteilhafterweise um einen Teil der Textillage herumgeführt, so dass eine Zugkraft, die auf das Zugelement wirkt, durch die Schlaufe auf die Textillage übertragen wird. Die beiden Enden des Fadens, die das distale Ende des Zugelementes bilden, werden vorteilhafterweise am distalen Ende des Prothesenliners aus der Textillage herausgeführt. Soll der Prothesenliner an individuelle Gegebenheiten eines Amputationsstumpfes angepasst werden, kann beispielsweise durch Ausübung einer Zugkraft auf die distalen Enden der Zugelemente eine Spannung und damit eine Verformung auf die Textillage und damit auf den daran oder umgebend befestigten Grundkörper ausgeübt werden. Auf diese Weise ist eine Individualisierung des Prothesenliners in zumindest beschränktem Umfang möglich.

Vorzugsweise werden die Zugelemente zumindest abschnittsweise innerhalb der Textillage geführt. Auf diese Weise wird einerseits verhindert, dass die Zugelemente sich zwischen ihrem distalen Ende und ihrem proximalen Ende ausschließlich auf der Außenseite der Textillage und damit gegebenenfalls auf der Außenlage des Prothesenliners befinden. In diesem Fall bestünde die Gefahr, dass beispielsweise der Benutzer des Prothesenliners beim Anlegen des Liners in die so gebildeten Schlaufen einhakt und aufgrund der dann wirkenden Kräfte Beschädigungen hervorruft. Durch das Führen der Zugelemente zumindest abschnittsweise innerhalb der Textillage können auch weitere Verbindungen zwischen dem jeweiligen Zugelement und der Textillage hervorgerufen werden, so dass auf das Zugelement wirkende Zugkräfte nicht nur an dessen proximalen Ende auf die Textillage übertragen werden können, sondern gegebenenfalls auch an Zwischenpunkten, die sich zwischen dem proximalen Ende und dem distalen Ende der Zugelemente befinden.

Besonders bevorzugt verfügt der Prothesenliner über eine distale Kappe mit wenigstens einem Befestigungselement zum Befestigen des Prothesenliners an einem Prothesenschaft, wobei die distalen Enden der Zugelemente mit der distalen Kappe verbunden, bevorzugt verklebt oder angespritzt sind. Bevor die distalen Enden der Zugelemente mit der distalen Kappe verbunden werden, wird vorteilhafterweise in der bereits beschriebenen Weise der Zug auf die distalen Enden der Zugelemente ausgeübt, um eine Anform- und Anpassung des Abschnittes des Prothesenliners an die individuellen Gegebenheiten des Benutzers des Prothesenliners vorzunehmen. Nachdem diese Einstellungen vorgenommen wurden, können die distalen Enden der Zugelemente mit der distalen Kappe verbunden, beispielsweise verklebt oder eingegossen werden, so dass sie unlösbar verbunden sind und eine spätere, insbesondere versehentliche oder fehlerhafte Änderung ausgeschlossen bleibt. Zudem wird auf diese Weise eine besonders gute Kraftübertragung zwischen der distalen Kappe, auf die die Zugkräfte beispielsweise in der Schwungphase eines Schrittes wirken, und den Zugelementen gewährleistet.

Bevorzugt ist die Kappe in Prothesenliner integriert, vorzugsweise in das elastische Linermaterial eingegossen oder eingespritzt.

Vorzugsweise ist der Prothesenliner ausgebildet, in einem angelegten Zustand ein Gelenk eines Amputationsstumpfes, insbesondere ein Knie oder einen Ellenbogen, zu umgeben, wobei der Abschnitt distal des Gelenks endet. Der Abschnitt der Textillage, in dem sich die Zugelemente befinden, ist bevorzugt folglich vollständig distal des jeweiligen Gelenkes angeordnet. Dadurch wird gewährleistet, dass die Elastizität entlang der Längserstreckung des Liners im Bereich des jeweiligen Gelenks, insbesondere des Knies, vorhanden ist, um die nötige Dehnung, die beim Beugen des jeweiligen Gelenkes notwendigerweise entsteht, aufbringen zu können.

Insbesondere aber nicht ausschließlich für den Fall, dass sich der Abschnitt mit den Zugelementen nicht ausschließlich distal eines Gelenkes des Trägers befindet, ist es von Vorteil, wenn die Zugelemente zumindest in dem Bereich des Gelenkes medial und/oder lateral des Gelenkes, insbesondere in den neutralen Fasern befinden. In einer bevorzugten Ausgestaltung sind die Zugelemente distal des Gelenkes und gegebenenfalls auch proximal des Gelenkes äquidistant über den Umfang und im Bereich des Gelenkes medial und/oder lateral des Gelenkes angeordnet sind.

Die Erfindung löst die gestellte Aufgabe zudem durch ein System aus einem Prothesenliner nach einer der hier beschriebenen Ausführungsformen und einem Prothesenschaft, wobei der Prothesenschaft derart ausgebildet ist, dass er mit dem Verbindungsbereich des Prothesenliners verbindbar ist

Mithilfe der beiliegenden Figuren werden nachfolgend einige Ausführungsbeispiele der vorliegenden Erfindung näher erläutert.

Es zeigen:
- Figuren 1 bis 3: - verschiedene Ausführungsbeispiele eines Prothesenliners, jeweils in einer Frontal- und einer Seitenansicht,
- Figur 4: - die schematische Darstellung eines für einen derartigen Liner verwendbaren Gestrickes und
- Figur 5: - ein weiteres Ausführungsbeispiel eines Prothesenliners in einer Frontal- und einer Seitenansicht.

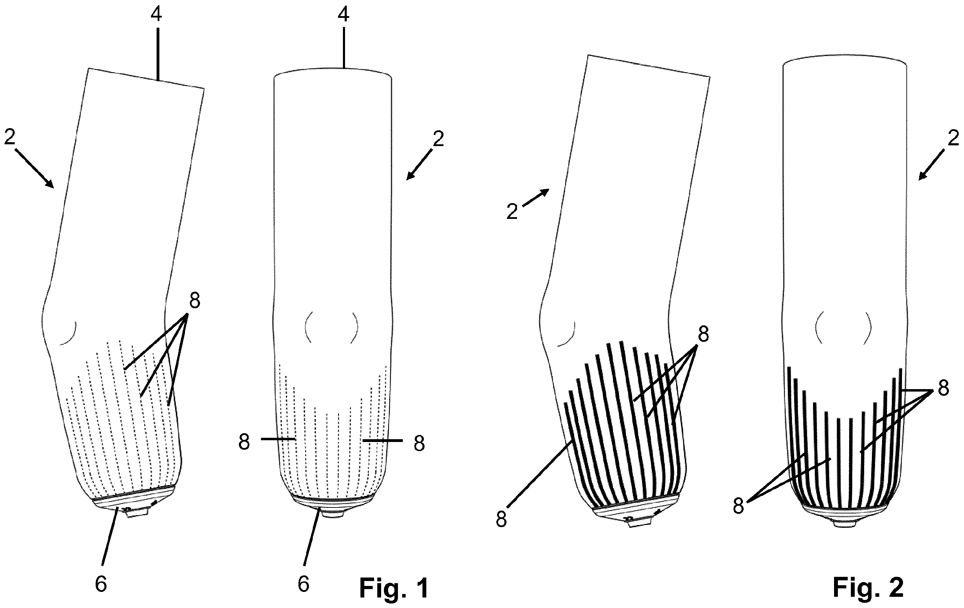

Figur 1 zeigt einen Prothesenliner 2 mit einem Grundkörper 4, an dessen distalem Bereich sich eine nicht explizit dargestellte Textillage befindet. Der Prothesenliner 2 verfügt über eine Anschlusskappe 6, an der Befestigungselemente angeordnet werden können, um den Prothesenliner 2 in einem nicht dargestellten Prothesenschaft anzuordnen.

In der bi-elastischen Textillage, die an dem Grundkörper 4 des Liners 2 angeordnet ist, sind mehrere Zugelemente 8 angeordnet, die als gestrichelte Linien dargestellt sind. Es handelt sich um Stehfäden, die in das Textil der Textillage eingestickt worden sind. Sie sind vorzugsweise aus einem inelastischen Material hergestellt oder aus einem Material hergestellt, das eine geringere Elastizität als die Textillage aufweist.

Figur 2 zeigt eine andere Ausgestaltung des Prothesenliners 2, bei dem wieder am distalen Bereich eine nicht dargestellte Textillage angeordnet ist. Anstelle der eingestickten Stehfäden aus Figur 1 sind in Figur 2 Zugelemente 8 in die Textillage eingebracht worden, die eine thermoplastische Beschichtung oder Schmelzfäden aufweisen oder selbst aus einem thermoplastischen Material hergestellt sind. Nachdem die Zugelemente 8 in Form der Stehfäden in das textile Material der Textillage eingebracht wurden, können sie durch erhöhende Temperatur aufgeschmolzen oder zumindest soweit aufgeweicht werden, dass sie sich mit der Textillage verbinden. Die Besonderheit besteht insbesondere darin, dass diese Verbindung nicht auf der Außenseite der Textillage, sondern innerhalb der Textillage erfolgt, da die Stehfäden zunächst in der Textillage integriert und in diese eingearbeitet wurden.

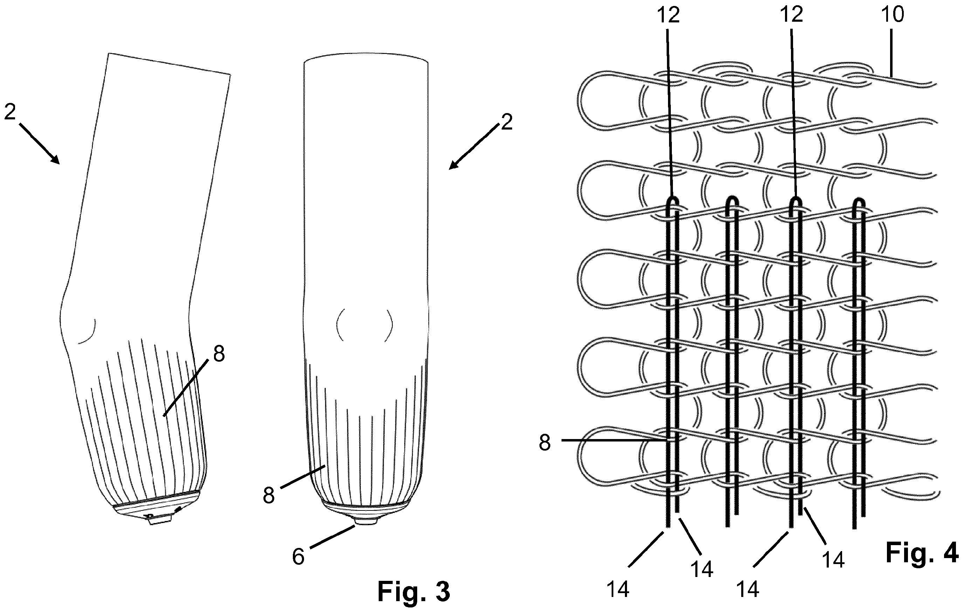

Figur 3 zeigt eine weitere Ausgestaltung des Prothesenliners 2, bei dem die Zugelemente 8 in Form von Stehfäden in das Textil der nicht dargestellten Textillage eingebracht sind.

Figur 4 zeigt schematisch, wie die Zugelemente 8 im Gestrick positioniert sind. Man erkennt das Gestrick 10, das einen Teil der elastischen Textillage bildet, die an oder in dem Linermaterial des Prothesenliners 2 angeordnet ist. Die Schlaufen des Gestrickes 10 werden aus einem Garn aus einem ersten Material oder einem Materialmix gebildet. Das Gestrick 10 verfügt in beiden Richtungen, in Figur 4 also sowohl nach oben und unten als auch nach rechts und links, über eine Elastizität, die es zu einem bielastischen Textil macht. Die Zugelemente 8 in Form der Stehfäden werden vom distalen Ende aus in das Gestrick eingebracht und verlaufen nach distal bis sie eine Umlenkschlaufe 12 bilden und parallel zum bisherigen Weg wieder in Richtung distal umgelenkt werden. Am distalen Ende können die beiden Enden miteinander verbunden, beispielsweise verklebt oder mit der distalen Anschlusskappe 6 verbunden werden.

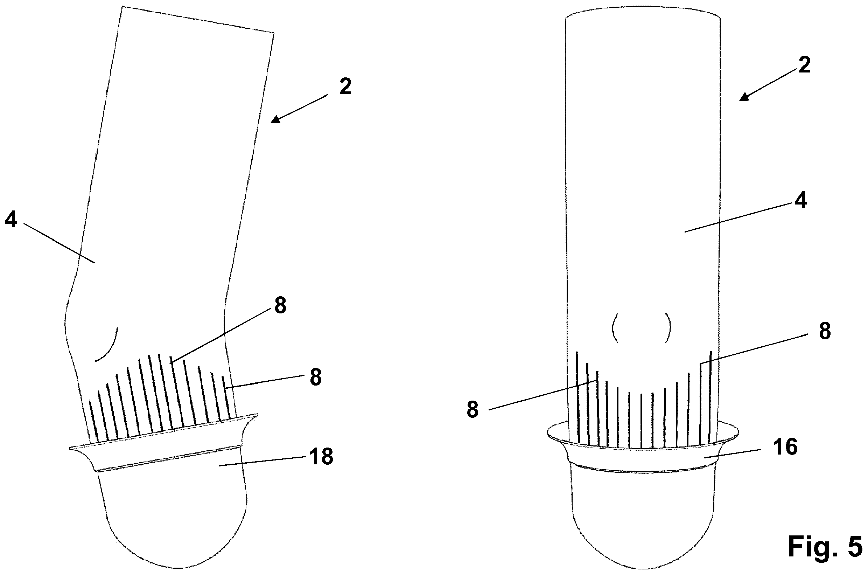

Figur 5 zeigt in einer Frontal- und einer Seitenansicht den Prothesenliner 2, dessen Grundkörper 4 eine Mehrzahl von Zugelementen 8 aufweist, die sich jedoch nicht bis zu dem distalen Ende des Prothesenliners 2 erstrecken. Der dargestellte Prothesenliner 2 verfügt über eine Dichtlippe 16, die mit einer Innenwand eines Prothesenschaftes n Kontakt kommt, wenn der Prothesenliner 2 im angelegten Zustand in den Prothesenschaft eingeführt wird. Dadurch wird distal der Dichtlippe 16, in den gezeigten Darstellungen also unterhalb der Dichtlippe 16, ein Volumen luftdicht abgeschlossen, in dem ein Unterdruck erzeugbar ist, durch den der Prothesenschaft an dem Prothesenliner 2 gehalten wird. In diesem Bereich distal der Dichtlippe 16 erstreckt sich der Verbindungsbereich 18.

### Bezugszeichenliste:

- 2: Prothesenliner
- 4: Grundkörper
- 6: Anschlusskappe
- 8: Zugelement
- 10: Gestrick
- 12: Umlenkschlaufe
- 14: Ende
- 16: Dichtlippe
- 18: Verbindungsbereich

## Patentansprüche

1. Prothesenliner (2) mit
- einem Grundkörper (4) aus einem elastischen Linermaterial, der
∘ ein offenes proximales Ende,
∘ ein dem proximalen Ende entgegengesetztes geschlossenes distales Ende und
o eine Längserstreckung aufweist, die sich von dem proximalen Ende zu dem distalen Ende erstreckt,
- einer bi-elastische Textillage, die an oder in dem Linermaterial angeordnet ist, und
- einem Verbindungsbereich (18), der angeordnet und eingerichtet ist, mit einem Prothesenschaft verbunden zu werden,
**dadurch gekennzeichnet, dass**
die Textillage in einem Abschnitt, der sich von dem Verbindungsbereich (18) nach proximal erstreckt, wenigstens ein Zugelement (8) aufweist, durch das die Elastizität der Textillage in Längsrichtung des Grundkörpers (4) reduziert ist und das Fasern und/oder Fäden aus einem inelastischen Material aufweist.

2. Prothesenliner (2) nach Anspruch 1, **dadurch gekennzeichnet, dass** sich der Abschnitt von dem distalen Ende ausgehend erstreckt, wobei der Verbindungsbereich (18) durch eine distale Kappe (6) mit wenigstens einem Befestigungselement zum Befestigen des Prothesenliners (2) an einem Prothesenschaft gebildet wird.

3. Prothesenliner (2) nach Anspruch 1 oder 2, **dadurch gekennzeichnet, dass** der Verbindungsbereich (18) proximal durch wenigstens eine Dichtlippe (16) begrenzt wird.

4. Prothesenliner (2) nach einem der vorstehenden Ansprüche, **dadurch gekennzeichnet, dass** sich das wenigstens eine Zugelement (8) in den Verbindungsbereich (18) hinein erstreckt und/oder die Textillage zusätzlich auch in dem Verbindungsbereich (18) wenigstens ein Zugelement (8) aufweist, durch das die Elastizität der Textillage in Längsrichtung des Grundkörpers (4) reduziert wird.

5. Prothesenliner (2) nach einem der vorstehenden Ansprüche, **dadurch gekennzeichnet, dass** die Textillage eine Mehrzahl von Zugelementen (8) aufweist, die vorzugsweise äquidistant über einen Umfang der Textillage verteilt angeordnet sind.

6. Prothesenliner (2) nach einem der vorstehenden Ansprüche, **dadurch gekennzeichnet, dass** die Zugelemente (8) an ihrem proximalen Ende, das bevorzugt von dem proximalen Ende des Grundkörpers (4) beabstandet ist, an der Textillage derart befestigt sind, dass Zugkräfte, die auf die Zugelement (8) in Längsrichtung wirken, auf die Textillage übertragen werden.

7. Prothesenliner (2) nach Anspruch 6, **dadurch gekennzeichnet, dass** die Zugelemente (8) an ihrem proximalen Ende eine Schlaufe (12) bilden, die um wenigstens ein Element der Textillage herumgeführt ist.

8. Prothesenliner (2) nach einem der vorstehenden Ansprüche, **dadurch gekennzeichnet, dass** die Zugelemente (8) zumindest abschnittsweise innerhalb der Textillage verlaufen.

9. Prothesenliner (2) nach einem der vorstehenden Ansprüche, **dadurch gekennzeichnet, dass** der Prothesenliner (2) eine distale Kappe (6) mit wenigstens einem Befestigungselement zum Befestigen des Prothesenliners (2) an einem Prothesenschaft aufweist, wobei die distalen Enden der Zugelemente (8) mit der distalen Kappe (6) verbunden, bevorzugt verklebt oder angespritzt sind.

10. Prothesenliner (2) nach Anspruch 9, **dadurch gekennzeichnet, dass** die distale Kappe (6) in den Prothesenliner (2) integriert, vorzugsweise in das elastische Linermaterial eingegossen oder eingespritzt ist.

11. Prothesenliner (2) nach einem der vorstehenden Ansprüche, **dadurch gekennzeichnet, dass** der Prothesenliner (2) ausgebildet ist, in einem angelegten Zustand ein Gelenk eines Amputationsstumpfes, insbesondere ein Knie oder einen Ellenbogen, zu umgeben, wobei der Abschnitt distal des Gelenkes endet.

12. System aus einem Prothesenliner (2) nach einem der vorstehenden Ansprüche und einem Prothesenschaft, wobei der Prothesenschaft derart ausgebildet ist, dass er mit dem Verbindungsbereich (18) des Prothesenliners (2) verbindbar ist.

## Claims

1. A prosthesis liner (2) with
- a base body (4) made of an elastic liner material which comprises
∘ an open proximal end,
∘ a closed distal end opposite the proximal end, and
∘ a longitudinal extension, which extends from the proximal end to the distal end,
- a bi-elastic textile layer arranged on or in the liner material, and
- a connection region (18) that is arranged and configured to be connected to a prosthesis socket,
**characterized in that**
the textile layer has, in a section that extends proximally from the connection region (18), at least one tension element (8) by means of which the elasticity of the textile layer in the longitudinal direction of the base body (4) is reduced and which has fibers and/or threads made of an inelastic material.

2. The prosthesis liner (2) according to claim 1, **characterized in that** the section extends from the distal end, wherein the connection region (18) is formed by a distal cap (6) with at least one fastening element for fastening the prosthesis liner (2) to a prosthesis socket.

3. The prosthesis liner (2) according to claim 1 or 2, **characterized in that** the connection region (18) is limited proximally by at least one sealing lip (16).

4. The prosthesis liner (2) according to one of the preceding claims, **characterized in that** the at least one tension element (8) extends into the connection region (18) and/or the textile layer also features at least one tension element (8) in the connection region as well, by way of which the elasticity of the textile layer is reduced in the longitudinal direction of the base body (4).

5. The prosthesis liner (2) according to one of the preceding claims, **characterized in that** the textile layer features a plurality of tension elements (8) which are preferably distributed equidistantly across a circumference of the textile layer.

6. The prosthesis liner (2) according to one of the preceding claims, **characterized in that** the tension elements (8) are fastened at their proximal end, which is preferably located at a distance from the proximal end of the base body (4), to the textile layer in such a way that tension forces acting on the tension element (8) in the longitudinal direction are transmitted to the textile layer.

7. The prosthesis liner (2) according to claim 6, **characterized in that** the tension elements (8) form a loop (12) at their proximal end, which is guided around at least one element of the textile layer.

8. The prosthesis liner (2) according to one of the preceding claims, **characterized in that** the tension elements (8) extend at least in sections within the textile layer.

9. The prosthesis liner (2) according to one of the preceding claims, **characterized in that** the prosthesis liner (2) has a distal cap (6) with at least one fastening element for fastening the prosthesis liner (2) to a prosthesis socket, wherein the distal ends of the tension elements (8) are bonded, preferably glued or moulded, to the distal cap (6).

10. The prosthesis liner (2) according to claim 9, **characterized in that** the distal cap (6) is integrated into the prosthesis liner (2), preferably cast or injected into the elastic liner material.

11. The prosthesis liner (2) according to one of the preceding claims, **characterized in that** the prosthesis liner (2) is designed to enclose a joint, especially a knee or an elbow, of an amputation stump when in the mounted state, with the section terminating distally of the joint.

12. A system composed of a prosthesis liner (2) according to one of the preceding claims and a prosthesis socket, the prosthesis socket being designed in such a way that it can be connected to the connection region (18) of the prosthesis liner (2).

## Revendications

1. Doublure prothétique (2) comportant
- un corps de base (4) en un matériau de doublure élastique, présentant
• une extrémité proximale ouverte,
• une extrémité distale fermée, opposée à l'extrémité proximale, et
• une extension longitudinale qui s'étend de l'extrémité proximale à l'extrémité distale,
- une couche textile bi-élastique disposée sur ou dans le matériau de doublure, et
- une zone de liaison (18) disposée et adaptée pour être reliée à une emboîture de prothèse,
**caractérisée en ce que**
la couche textile présente, dans une portion qui s'étend vers le côté proximal à partir de la zone de liaison (18), au moins un élément de traction (8) par lequel l'élasticité de la couche textile est réduite dans la direction longitudinale du corps de base (4) et qui comprend des fibres et/ou des fils en un matériau inélastique.

2. Doublure prothétique (2) selon la revendication 1,
**caractérisée en ce que** la portion s'étend à partir de l'extrémité distale, la zone de liaison (18) étant formée par un capuchon distal (6) présentant au moins un élément de fixation pour fixer la doublure prothétique (2) à une emboîture de prothèse.

3. Doublure prothétique (2) selon la revendication 1 ou 2,
**caractérisée en ce que** la zone de liaison (18) est délimitée du côté proximal par au moins une lèvre d'étanchéité (16).

4. Doublure prothétique (2) selon l'une des revendications précédentes,
**caractérisée en ce que** ledit au moins un élément de traction (8) s'étend jusque dans la zone de liaison (18), et/ou la couche textile présente en supplément, également dans la zone de liaison (18), au moins un élément de traction (8) qui réduit l'élasticité de la couche textile dans la direction longitudinale du corps de base (4).

5. Doublure prothétique (2) selon l'une des revendications précédentes,
**caractérisée en ce que** la couche textile présente une pluralité d'éléments de traction (8), qui sont de préférence répartis de manière équidistante sur une périphérie de la couche textile.

6. Doublure prothétique (2) selon l'une des revendications précédentes,
**caractérisée en ce que** les éléments de traction (8) sont fixés à la couche textile, à leur extrémité proximale, de préférence espacée de l'extrémité proximale du corps de base (4), de manière à transmettre à la couche textile les forces de traction qui agissent sur les éléments de traction (8) dans la direction longitudinale.

7. Doublure prothétique (2) selon la revendication 6,
**caractérisée en ce que** les éléments de traction (8) forment à leur extrémité proximale une boucle (12) qui est passée autour d'au moins un élément de la couche textile.

8. Doublure prothétique (2) selon l'une des revendications précédentes,
**caractérisée en ce que** les éléments de traction (8) s'étendent au moins localement à l'intérieur de la couche textile.

9. Doublure prothétique (2) selon l'une des revendications précédentes,
**caractérisée en ce que** la doublure prothétique (2) comprend un capuchon distal (6) présentant au moins un élément de fixation pour fixer la doublure prothétique (2) à une emboîture de prothèse, les extrémités distales des éléments de traction (8) étant reliées au capuchon distal (6), de préférence par collage ou par moulage par injection.

10. Doublure prothétique (2) selon la revendication 9,
**caractérisée en ce que** le capuchon distal (6) est intégré dans la doublure prothétique (2), de préférence coulé ou injecté dans le matériau élastique de la doublure.

11. Doublure prothétique (2) selon l'une des revendications précédentes,
**caractérisée en ce que** la doublure prothétique (2) est conçue pour entourer, à l'état appliqué, une articulation d'un moignon d'amputation, en particulier un genou ou un coude, ladite portion se terminant du côté distal par rapport à l'articulation.

12. Système composé d'une doublure prothétique (2) selon l'une des revendications précédentes et d'une emboîture de prothèse, l'emboîture de prothèse étant conçue de manière à pouvoir être reliée à la zone de liaison (18) de la doublure prothétique (2).
